# EUROPEAN PATENT APPLICATION

(11) **EP 2 962 657 A1**
(43) Date of publication of application: **06.01.2016**
(21) Application number: 15001771.3
(22) Date of filing: 16.06.2015
(51) Int. Cl.: A61C 8/00, A61L 27/06, C25D 11/26

(54) **METHOD FOR APPLYING AN ANTIBACTERIAL PROTECTION TO A DENTAL IMPLANT, AND DENTAL IMPLANT OBTAINED**

(30) Priority: 04.07.2014 ES 201431007
(71) Applicant: Gil Mur Javier, 08022 Barcelona (ES); Vogul, S.L.U., Andorra La Vella (ES)
(72) Inventor: Rodriguez Rius, Daniel, Andorra La Vella (ES); Godoy Gallardo, Maria, Andorra La Vella (ES); Manero Planella, Jose Maria, Andorra La Vella (ES)
(74) Representative: Diaz Nunez, Joaquin

(57) **Abstract**

The procedure is applicable in a dental implant with at least one outer surface made of titanium or titanium alloy and comprises:- a prior preparation of the external surface of the implant: including at least one of the following operations: roughing, polishing, cleaning, decontamination and/or elimination of a layer of titanium oxide from said outer surface of the implant; and - the deposit of particles of silver on an outer surface of the implant, made of titanium or titanium alloy, through a pulsed current electrochemical anodization process. The invention includes the obtained dental implant with antibacterial protection.

## Description

### Object of the invention.

The object of the present invention is a method for applying an antibacterial protection to a dental implant, and more specifically to a dental implant formed at least externally of titanium or titanium alloy; said method having characteristics intended to produce a deposit of silver particles from nanometer to micrometer size on the outer surface of the implant, in order to protect them from microbial penetration and prevent diseases caused by bacteria, as it is the case of peri-implantitis.

It is worth mentioning that the deposit of silver particles may be done on the titanium surfaces both of the dental implant itself and in prosthetic part and in the connection screw of the same with the dental implant.

Therefore, in this invention the term "dental implant" should be interpreted in a broad sense, and may include both the implant itself and the prosthetic part of the same, and the connection screw.

This invention also includes the dental implant with antibacterial protection obtained according to the procedure in question.

### Field of application of the invention.

This invention is applicable in the sector dedicated to the manufacture of dental implants and prosthetic parts.

### State of the art.

One of the major drawbacks of dental implants is their behavior against penetration and action of the bacteria that can cause complications and various diseases such as peri-implantitis.

Any one of the solutions proposed to minimize this problem, especially in the stage after the incorporation of the implant in the body consists of applying on the Implant a coating or passivating surface against adhesion and/or bacterial growth.

Thus, for example, the document ES 2 246 431 T3 describes dental implants having bacterial resistance provided by a coating that is applied on at least part of a surface of the dental implant and wherein said coating contains a polymer consisting of an alkoxyl group substituted with at least one fluorine atom.

This blocompatible coating is applicable on implants of different materials such as plastics, metals, metal alloys and ceramics and biocompatible polymers.

In the mentioned background one of the drawbacks is determined by the degree of adhesion of the coating to the surface of the implant, especially taking into account that it can be made of different materials.

Therefore, the technical problem that arises is the development of a method for applying an antibacterial protection to an implant, specifically on implants of titanium or titanium alloys, that provides a permanent antibacterial protection, i.e. linked inseparably to the implant.

### Description of the Invention.

The method for applying an antibacterial protection to a dental implant, object of this invention, is applicable in implants comprising at least one outer surface made of titanium or titanium alloy; comprising said method:
a) a prior preparation of the external surface of the implant; including at least one of the following operations: roughing, polishing, cleaning, decontamination and/or elimination of a layer of titanium oxide from said outer surface of the implant, and
b) the deposit of particles of silver on an outer surface of the implant, made of titanium or titanium alloy, through a pulsed current electrochemical anodization process.

This procedure achieves the deposit and integration on the outer surface of the implant of silver particles providing such implant high antibacterial protection on a permanent basis.

According to the invention the pulsed current electrochemical anodization process Is carried out in an aqueous solution with silver nitrate (AgNO₃) and a coordination compound, preferably sodium thiosulfate (Na₂S₂O₃), using the titanium of the dental implant as a working electrode and a platinum foil as a counter electrode.

In order to optimize the aforementioned method the electrochemical anodization process comprises the application to the working electrode of a pulsed potential with a rectangular pulse waveform, and with a duration of a full cycle comprised between 20 and 40 seconds, and preferably between 20 and 30 seconds; said anodization process including the realization of a number of full cycles comprises between 300 and 700 and preferably between 400 and 600.

This invention also includes the dental implant with antibacterial protection obtained by the previously indicated method said implant being formed at least in its outer surface of titanium or titanium alloys and having the peculiarity of comprising silver particles from one nanometer to micrometer size deposited on the outer surface of the dental implant.

### Description of the drawings.

In order to complement the description that is being carried out and with the object to help to a better understanding of the characteristics of the invention, a set of drawings is accompanied to the present specification, in which, with an illustrative and non-limiting character, the following has been represented:
- Figure 1 shows an exemplary embodiment of the rectangular pulse waveform of the current used in the anodization process in a full cycle.
- Figures 2 and 3 show respective images observed on Focused Ion Beam electron microscopy of a portion of dental implant with antibacterial protection according to the Invention, with different magnifications and wherein the silver particles deposited electrochemically on titanium of the implant can be seen.

### Preferred embodiment of the invention.

In a specific example embodiment of the method of the invention it is initially carried out the roughing of the titanium outer surface of a dental implant using silicon carbide papers of decreasing grain size from 500 to 25 microns, being then polished with alumina particles in suspension of 1.0 micrometers and 0.05 micrometers.

Subsequently, the cleaning of the surface of the implant is carried out by a treatment with ethanol ultrasounds, distilled water and acetone over a period comprised between 5 and 30 minutes, and preferably 15 minutes each, finally said outer surfaces drying with gas argon.

Then the outer surface of the implant is pretreated for 10 seconds with an acidic bath [250 mL HNO₃ 60%, 17.5 g NH₄HF₂ and 250 mL ultrapure water] to eliminate the contamination of the surface and the possible layer of titanium oxide existing on the outer surface of the implant; leaving the outer surface of the implant ready for the deposit of silver particles thereon.

This process is based on the use of a pulse anodization method to transfer silver coordinated complex with negative charge on the surface of the titanium.

The titanium is roughed with silicon carbide papers of decreasing grain size, and polished with alumina particles in suspension (1.0 µm and 0.05 µm). Surfaces are treated with ethanol ultrasounds, distilled water and acetone during 15 minutes each, and dried with argon gas.

Then the deposit of silver particles on the outer surface of the implant is carried out through a electrochemical anodization process in aqueous solution with silver nitrate (AgNO₃) and sodium thiosulfate (Na₂S₂O₃) at room temperature and magnetic stirring, with a concentration ratio of 0.1 M:0.2 M AgNO₃:Na₂S₂O₃.

In this process, the titanium is used as a working electrode and a platinum foil as a counter electrode.

The formation of silver complexes follows the reaction:

Ag⁺ + 2(S₂O₃)²⁻ ↔ [Ag(S₂O₃)²]³⁻

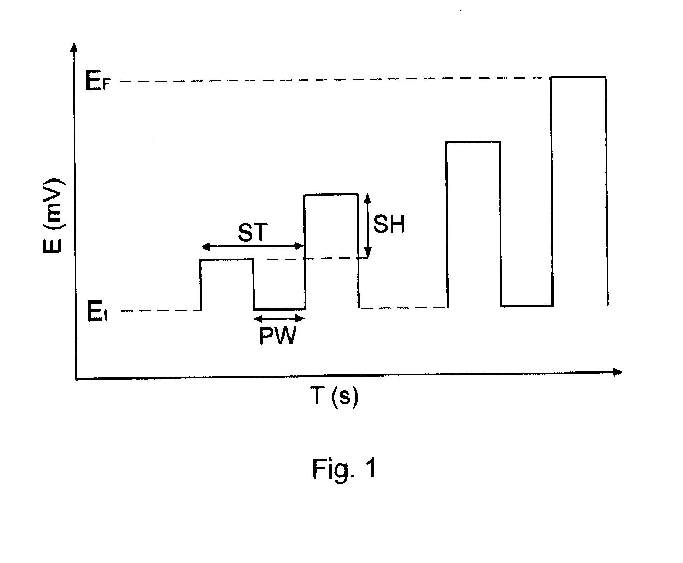

The anodization process is controlled by a potentiostat; applying to the working electrode a pulsed potential with a rectangular pulse waveform as shown in the Figure 1 and with the following values:
(E_{I}= 0 V, E_{F}= 5 V, ST = 500 ms, SH = 10 mV, PW = 100 ms).

Being E_{I} the initial voltage, E_{F} the final voltage, ST the period, SH the increase in pulse amplitude and PW the break time.

The application of pulsed potential is carried out in cycles of 25 seconds, the electrochemical anodization process including a total of 500 full cycles.

After treatment, the surfaces are cleaned with ultrasound in ethanol bath, distilled water and acetone during 15 minutes each.

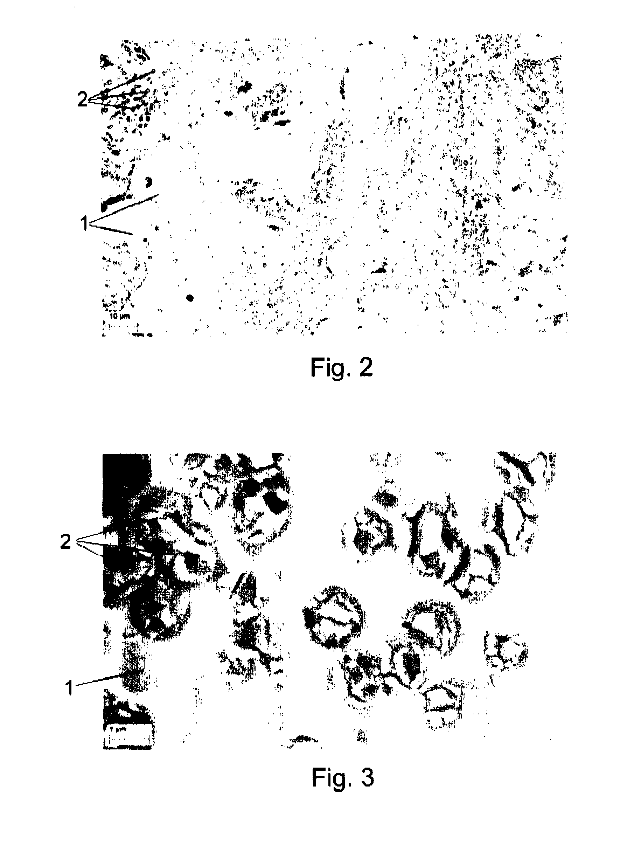

In Figures 2 and 3 can be seen on Focused Ion Beam electron microscopy the sliver particles (2) remaining on the titanium surface of the implant (1).

These silver particles (2) not affect the osseointegration of the implant and protect it from bacterial attack.

The trials of bacterial plaque cultures have shown a great reduction of bacterial plaques in strains such as *Streptococcus sanguinis* and *Lactobacillus salivarius,* without the biocompatibility or biological behavior of fibroblastic cells are affected. Furthermore, the release of silver ions to the physiological medium can not be detected.

Having sufficiently described the nature of the invention as well as a preferred embodiment thereof, it is stated for all intents and purposes that the materials, shape, size and arrangement of the elements described may be modified provided this does not entail altering the essential features of the invention which are claimed below.

## Claims

1. Method for applying an antibacterial protection to a dental implant; comprising said implant at least one outer surface made of titanium or titanium alloy; **characterized in that** it comprises:
a) a prior preparation of the external surface of the implant; including at least one of the following operations: roughing, polishing, cleaning, decontamination and/or elimination of a layer of titanium oxide from said outer surface of the implant, and
b) the deposit of particles of silver on an outer surface of the implant, made of titanium or titanium alloy, through a pulsed current electrochemical anodization process.

2. Method according to claim 1, **characterized in that** the pulsed current electrochemical anodization process is carried out in an aqueous solution with silver nitrate (AgNO₃) and a coordination compound, preferably sodium thiosulfate (Na₂S₂O₃)]; using the titanium of the dental implant as a working electrode and a platinum foil as a counter electrode.

3. Method according to claim 2, **characterized in that** the anodization process comprises the application to the working electrode of a pulsed potential with a rectangular pulse waveform with a duration of a full cycle comprised between 10 and 40 seconds, the anodization process including the realization of a number of full cycles comprises between 200 and 700, and preferably between 400 and 600.

4. Method according to claim 1, **characterized in that** the prior preparation of the external surface of the implant comprises roughing said surface with silicon carbide papers of decreasing grain size from 500 µm to 25 µm.

5. Method according to claim 1, **characterized in that the** prior preparation of the external surface of the implant comprises polishing said surface with a alumina particles in suspension from 1.0 µm to 0,05 µm.

6. Method according to claim 1, **characterized in that** the cleaning the outer surface of the implant comprises treating said surface with ethanol ultrasounds, distilled water and acetone during 15 minutes each, and its subsequent drying with argon gas.

7. Method according to claim 1, **characterized in that** decontamination and/or elimination of the layer of titanium oxide of the outer surface of the implant comprises its treatment over a period from 5 to 30 seconds with an acidic bath containing 250 mL HNO₃ 60%, from 5 to 30 g NH₄HF₂ and from 100 to 500 mL ultrapure water.

8. Dental implant with antibacterial protection; the implant of which is formed, at least In its outer surface, of titanium or titanium alloys, **characterized in that** it comprises silver particles from a nanometer to micrometer size deposited on the outer surface of the dental implant.
